# EUROPÄISCHE PATENTSCHRIFT

(11) **EP 1 230 549 B1**
(45) Veröffentlichungstag und Bekanntmachung des Hinweises auf die Patenterteilung: **11.05.2005**
(21) Anmeldenummer: 00987251.6
(22) Anmeldetag: 15.11.2000
(51) Int. Cl.: G01N 33/533, G01N 33/58

(54) **VERFAHREN ZUR SOLUBILISIERUNG VON OPTISCHEN MARKERN**
METHOD FOR SOLUBILISING OPTICAL MARKERS
PROCEDE DE SOLUBILISATION DE MARQUEURS OPTIQUES

(30) Priorität: 16.11.1999 DE 19954934
(43) Veröffentlichungstag der Anmeldung: 14.08.2002
(73) Patentinhaber: Chromeon GmbH, 93053 Regensburg (DE)
(72) Erfinder: WOLFBEIS, Otto, 93051 Regensburg (DE); LEHMANN, Frank, 07749 Jena (DE); ARBTER, Michaela, 84036 Kumhausen-Hachelstuhl (DE)
(74) Vertreter: Dey, Michael, Dr.
(86) Internationale Anmeldenummer: PCT/EP2000/011310
(87) Internationale Veröffentlichungsnummer: WO 2001/036973

(56) Entgegenhaltungen:
- EP-A- 0 502 723
- WO-A-84/03698
- WO-A-88/04777
- WO-A-91/16344
- DE-A- 4 439 347

## Beschreibung

Optische Marker werden für diagnostische Assays benötigt. Unter den optischen Markern sind Fluoreszenzmarker besonders vorteilhaft, da sie eine Vielfalt von Möglichkeiten der Informationsgewinnung erlauben, z. B. über die Messung der Intensität, der Abklingzeit, der Polarisation oder des Energietransfers, und zusätzlich eine hohe Empfindlichkeit bzw. niedrige Nachweisgrenze gestatten. Die Markierung erfolgt durch kovalente Konjugation an Proteine, Peptide, Oligomere, DNA, RNA oder Arzneistoffe. Die kovalente Konjugation zwischen optischem Marker und Molekül erfolgt mittels einer reaktiven Gruppe, die meist aus folgenden Funktionalitäten ausgewählt wird: Isothiocyanate, Isocyanate, Monochlortriazine, Dichlortriazine, Aziridine, Sulfonylhalogenide, N-Hydroxysuccinimidester, Imido-Ester, Glyoxal oder Aldehyd für Amin- und Hydroxy-Funktionen, bzw. Maleimide oder Iodacetamide für Thiol-Funktionen, sowie Phosphoramidite für die Markierung der DNA oder RNA oder deren Bruchstücke. Diese Konjugation resultiert in einer kovalenten Bindung zwischen Marker und Molekül.

Für die Analytik wasserlöslicher Substanzen, z. B. der meisten Biomoleküle, ist es erforderlich, dass die Marker in wässriger Lösung zur Anwendung gebracht werden können. Mit zunehmender Molekülgröße werden aber Chromophore immer weniger wasserlöslich. Um deren Wasserlöslichkeit zu verbessern, werden solubilisierende Gruppen in die Chromophore eingeführt, z. B. Carbonsäuren (-COOH) oder Sulfonsäuren (-SO₃H) bzw. deren Salze. Die COOH- bzw. SO₃H-Gruppen können dabei entweder direkt oder über einen Spacer an das Chromophor des optischen Markers gebunden sein. Die Nachteile dieser Verbindungen liegen in deren aufwendiger Synthese und deren schwierigen Aufreinigung.

Aufgabe der vorliegenden Erfindung war es, ein Verfahren zur Solubilisierung zur Verfügung zu stellen, das die oben genannten Nachteile nicht besitzt.

Erfindungsgemäß erhält man wasserlösliche Marker dadurch, dass im Verlaufe der Synthese Phosphorsäurereste oder Phosphonsäurereste bzw. deren Salze oder Monoester direkt oder über eine Alkylkette in den Chromophor eingebaut werden. Die Verfahren zur Einführung derartiger Gruppen in organische Verbindungen sind beschrieben (Houben/Weyl, Methoden der organischen Chemie, 4. Auflage, Band XII/1 und 2, Organische Phosphorverbindungen) Typische Strukturen derartiger Verbindungen sind im Folgenden dargestellt (I-XV):

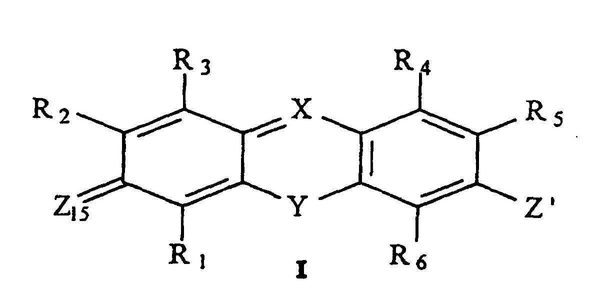

In den Verbindungen I kann stehen:
- X für C-R₇ oder N;
- Y für NR₈, O, S oder C(R₉)(R₁₀);
- Z für O oder N(R₁₁)(R₁₂)⁺;
- Z' für OH oder N(R₁₃)(R₁₄);
- R₁ bis R₁₄ für H, Alkyl, Cycloalkyl, Aryl, Teile eines aromatischen, heterocyclischen oder gesättigten, auch substituierten Ringsystems (auch in Verbindung mit Z oder Z'), Alkoxy, Hydroxy, Nitro, Halogen, N(R₁₅)(R₁₆) mit R₁₅ und R₁₆ von der gleichen Beschaffenheit wie R₁ bis R₁₄, SR₁₇ mit R₁₇ von der gleichen Beschaffenheit wie R₁ bis R₁₄;
- zumindest einer der Substituenten R₁ bis R₁₇ für eine reaktive Gruppe, welche eine kovalente Verknüpfung des Farbstoffes mit einem Biomolekül oder Arzneistoff ermöglicht. Die reaktive Gruppe kann aus folgenden Funktionalitäten ausgewählt werden: Isothiocyanate, Isocyanate, Monochlortriazine, Dichlortriazine, Aziridine, Sulfonylhalogenide, N-Hydroxysuccinimidester, Imido-Ester, Glyoxal oder Aldehyd für Amin- und Hydroxy-Funktionen bzw. Maleimide oder Iodacetamide für Thiol-Funktionen sowie Phosphoramidite für die Markierung der DNA oder RNA oder deren Bruchstücke. Diese reaktiven Gruppen können auch über lineare oder verzweigte Spacer-Gruppen der allgemeinen Summenformeln -(CH₂)ₙ-, -[(CH₂)ₘ-O-(CH₂)ₙ]- am eigentlichen Chromophor gebunden sein, wobei n und m Werte von 1 bis 18 annehmen können;
- R₁ bis R₁₆ für einen ionisierten, die Wasserlöslichkeit verbessernden Substituenten aus der Gruppe SO₃⁻, COO⁻, PO₃²⁻, OPO₃²⁻, PO₃R⁻, OPO₃R⁻oder NR₃⁺, wobei mindestens eine dieser Gruppen PO₃²⁻, OPO₃²⁻, PO₃R⁻ oder OPO₃R⁻ ist.

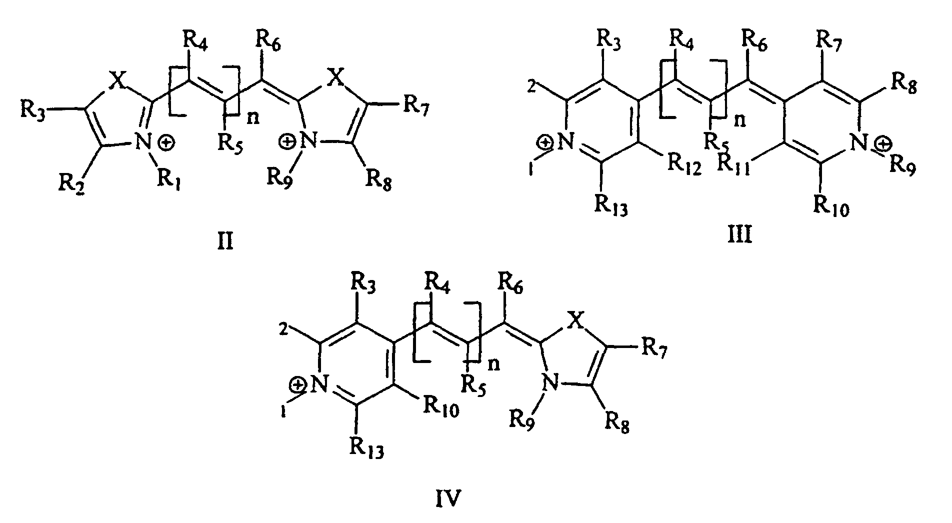

In den Verbindungen II bis IV kann stehen:
- X für C(R₁₄)(R₁₅), N, O, S, Se oder CH=CH;
- n für die Zahlenwerte 1, 2 oder 3;
- R₁ bis R₁₅ für H, Alkyl, Cycloalkyl, Aryl, Teile eines aromatischen, heterocyclischen oder gesättigten, auch substituierten Ringsystems (auch in Verbindung mit Z oder Z'), Alkoxy oder Hydroxy;
- die Substituenten R₄ und R₅ auch für eine Verbrückung über vier-, fünf- und sechsgliedrige Ringsysteme, an denen sich auch reaktive Gruppen befinden können. Solche Ringverbrückungen der Polymethinkette sind im Folgenden gezeigt. So kann die Struktureinheit 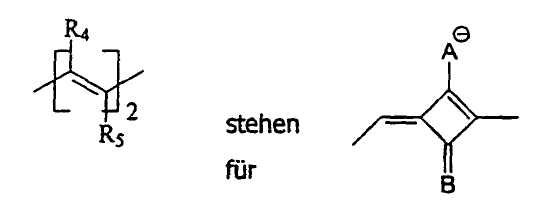 oder 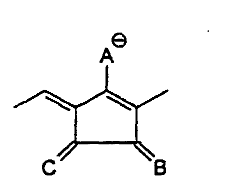 und die Struktureinheit 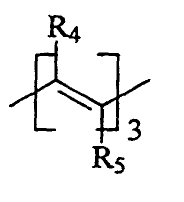 kann stehen für 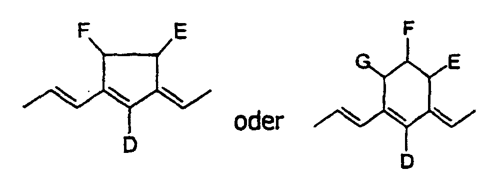 wobei die Substituenten **A, B, C, D, E, F** und **G** die gleichen Funktionalitäten wie R₁ bis R₁₅ besitzen können. Zusätzlich können **A, B** und **C** für O, S, C(CN)₂ bzw. N-R stehen, wobei R in N-R für einen aliphatischen oder aromatischen bzw. einem reaktiven aliphatischen oder aromatische Rest wie (CH₂)ₙCOOH oder (CH₂)ₙNH₂ steht, mit Werten von n zwischen 1 und 18. **D** kann weiterhin für Cl oder ein aromatisches bzw. aliphatisches Ringsystem stehen, an dem reaktive Substituenten analog zu R₁ bis R₁₅ angebracht sind;
- zumindest einer der Substituenten R₁ bis R₁₅ für eine reaktive Gruppe, welche eine kovalente Verknüpfung des Farbstoffes mit einem Biomolekül oder Arzneistoff ermöglicht Die reaktive Gruppe kann aus folgenden Funktionalitäten ausgewählt werden: Isothiocyanate, Isocyanate, Monochlortriazine, Dichlortriazine, Aziridine, Sulfonylhalogenide, N-Hydroxysuccinimidester, Imido-Ester, Glyoxal oder Aldehyd für Amin- und Hydroxy-Funktionen bzw. Maleimide oder Iodacetamide für Thiol-Funktionen sowie Phosphoramidite für die Markierung der DNA oder RNA oder deren Bruchstücke. Diese reaktiven Gruppen können auch über lineare oder verzweigte Spacer-Gruppen der allgemeinen Summenformeln -(CH₂)ₙoder -[(CH₂)ₘ-O-(CH₂)ₙ]ₒ- am eigentlichen Chromophor gebunden sein,
wobei n , m bzw. o Werte von 1 bis 18 annehmen können;
- R₁ bis R₁₄ für einen ionisierten, die Wasserlöslichkeit verbessernden Substituenten aus der Gruppe SO₃⁻, COO⁻, PO₃²⁻, OPO₃²⁻, PO₃R⁻, OPO₃R⁻oder NR₃⁺, wobei mindestens eine dieser Gruppen PO₃²⁻; OPO₃²⁻, PO₃R⁻ oder OPO₃R⁻ ist

### Ausführungsbeispiele

### 1-(2-Phosphonethyl)-2,3,3-trimethylindoleninium-5-sulfonat

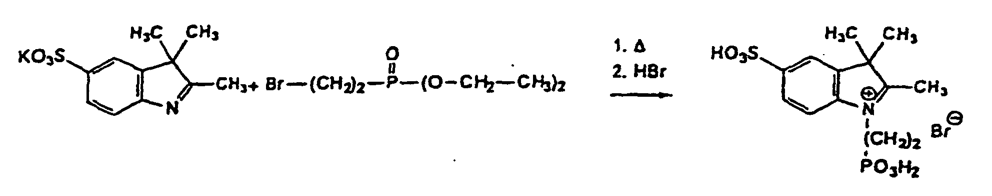

832 mg (3 mmol) des Kaliumsalzes von 2,3,3-Trimethylindolenin-5-sulfonat und 760 mg (3,1 mmol) Bromethylphosphonsäurediethylester werden im Druckrohr 20 h auf 130 °C erhitzt. Die Reaktionsmischung wird auf Raumtemperatur abgekühlt, mit 4 mL 47 %-iger HBr versetzt und 1,5 h auf 110 °C erhitzt. Das Lösungsmittel wird abrotiert und der Rückstand am Ölpumpenvakuum getrocknet. Der amorphe Rückstand wird mit 10 mL 2-Propanol versetzt, wobei das Produkt ausfällt. Nach Zugabe von 10 mL Ether wird das Produkt abgesaugt und im Exsikkator getrocknet.
Ausbeute: 769 mg (62%), rosa Pulver, C₁₃H₁₉BrNO₆PS (428,23 g/mol).

### 1-(2-Phosphonethyl)-4-methylchinolinium-bromid

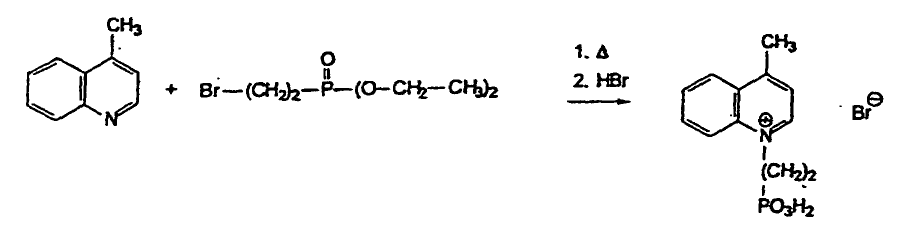

716 mg (5 mmol) Lepidin und 1,23 g (5 mmol) 2-Bromethanphosphonsäurediethylester werden im Druckrohr 18 h auf 100 °C erhitzt Das amorphe Produkt wird in 10 mL 47%-iger HBr gelöst und 2 h unter Rückfluß erhitzt. Anschließend wird das Lösungsmittel abrotiert Der Rückstand wird in Ethanol/Methanol gelöst und mit.Ethylacetat gefällt.
Ausbeute: 432 mg (26%), graues Pulver, C₁₂H₁₅BrNO₃P (332,13 g/mol).

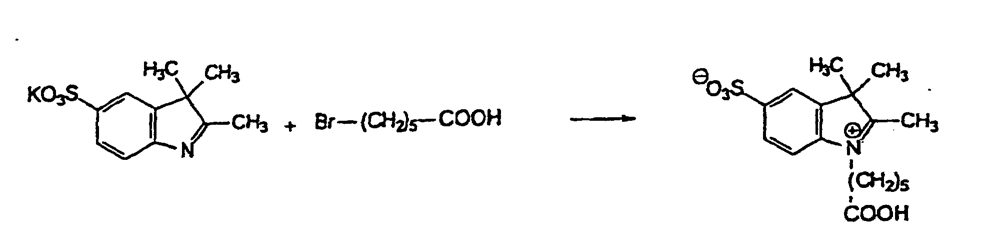

### 1-(5-Carboxypentyl)-2,3,3-trimethylindoleninium-5-sulfonat

15.9 g (57 mmol) des Kaliumsalzes von 2,3,3-Trimethylindolenin-5-sulfonat und 12.9 g (66 mmol) 6-Bromhexansäure werden in 150 mL 1,2-Dichlorobenzol 14 h unter Stickstoff refluxiert. Die Lösung wird auf Raumtemperatur abgekühlt und das Lösungsmittel abdekantiert. Das amorphe rosa Produkt wird mit einer 2-Propanol/Ether Mischung (1:1 v/v) versetzt und bei Raumtemperatur gerührt, bis das Produkt auskristallisiert. Das rosa Präzipitat wird mit Aceton, Chloroform und Ether gewaschen und im Exsikkator über P₂O₅ getrocknet.
Ausbeute: 15.8 g (78%), rosa Pulver, C₁₇H₂₃NO₅S (353.43 g/mol).

### 1-[1-[5-Carboxypentyl]-3,3-dimethyl-5-sulfo-2-indolinylidenmethyl]-2-butoxycyclo-buten-3,4-dion

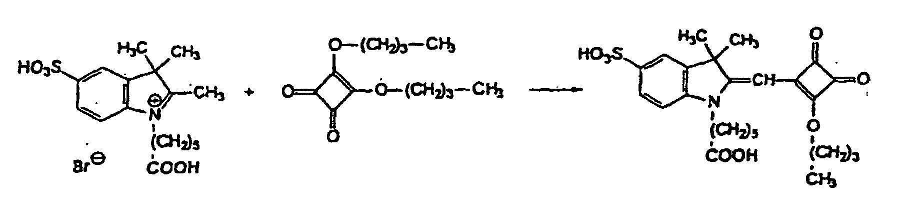

500 mg (1.4 mmol) 1-(5-Carboxypentyl)-2,3,3-trimethylindoleninium-5-sulfonat werden in 20 mL Ethanol gelöst Nach Zugabe von 1 mL Triethylamin werden 350 µL (1.6 mmol) Quadratsäuredibutylester langsam zugetropft. Die Mischung wird 2 h unter Rückfluß erhitzt. Nach dem Abkühlen wird das Lösungsmittel abrotiert. Der gelbbraune Rückstand wird in 20 mL Wasser gelöst und dreimal mit je 20 mL Chloroform extrahiert Die wässrige Phase wird gesammelt und das Wasser wird abrotiert. Der amorphe Rückstand wird mit Ether versetzt und bei Raumtemperatur über Nacht gerührt, bis das Produkt auskristallisiert. Das Rohprodukt wird aus 2-Butanoi umkristatlisiert
Ausbeute: 355 mg (50%), orangefarbenes Pulver, C₂₅H₃₁NO₈S (505.58 g/mol).

### Farbstoff 1

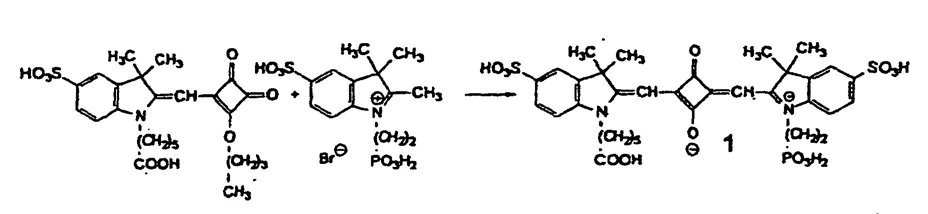

63 mg (0,125 mmol) 1-[1-[5-Carboxypentyl]-3,3-dimethyl-5-sulfo-2-indolinylidenmethyl]-2-butoxy-cyclobuten-3,4-dion und 54 mg (0,125 mmol) 1-(2-Phosphonethyl)-2,3,3-trimethyl-indoleninium-5-sul-fonat werden in 10 mL eines Gemischs aus Butanol und Toliren (1:1 v/v) 20 h auf 120 °C erhitzt. Nach dem Abkühlen wird das Lösungsmittel abrotiert. Der Rückstand wird in 10 mL 0,1 N HCl 2 h erhitzt, das Lösungsmittel anschließend abrotiert und das blaue Produkt mittels Chromatographie (RP-C18, Methanol/ Wasser 70/30 v/v) isoliert.
Ausbeute: 19 mg (20%), C₃₄H₃₉N₂O₁₃PS₂ (778,8 g/mol).

### Farbstoff 2

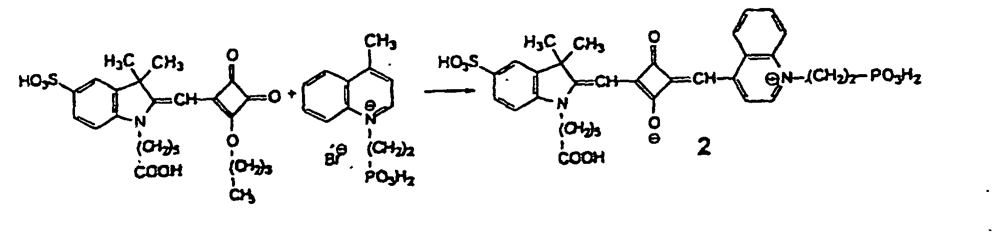

82 mg (0,15 mmol) 1-[1-[5-Carboxypentyl]-3,3-dimethyl-5-sulfo-2-indolinylidenmethyl]-2-butoxy-cyclobuten-3,4-dion und 48 mg (0,15 mmol) 1-(2-Phosphonethyl)-lepidinium-bromid werden in einem Gemisch aus Butanol und Toluen (1:1 v/v) 16 h auf 80 °C erhitzt. Nach dem Abkühlen wird das Lösungsmittel abrotiert. Der Rückstand wird in 10 mL 0,1 N HCl 2 h erhitzt, das Lösungsmittel anschließend abrotiert und das blaue Produkt mittels Chromatographie (RP-C18, Methanol/Wasser 70/30 v/v) isoliert.
Ausbeute: 32 mg (30%),C₃₅H₄₀N₂O₁₀PS (711,7 g/mol).

### Farbstoff 3

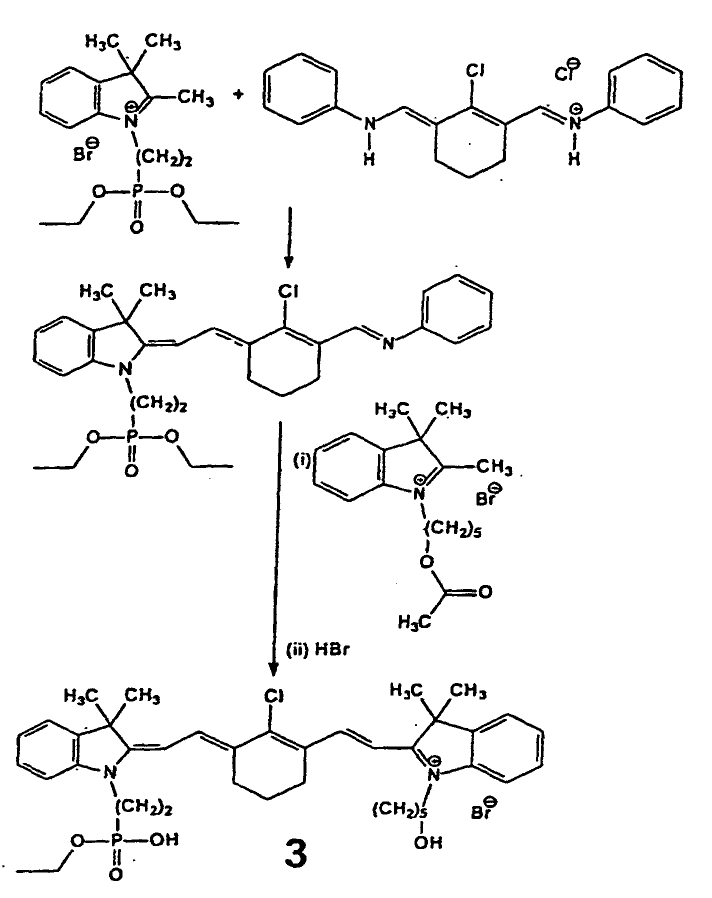

111 mg (0,275 mmol) 1-(2-Diethylphosphonethyl)-2,3,3-trimethylindoleniniumbromid, hergestellt analog zum 1-(2-Phosphonethyl)-2,3,3-trimethyl-indoleninium-5-sulfonat, jedoch ohne den Schritt der sauren Esterhydrolyse, werden zusammen mit 89,8 mg (0,25 mmol) N-[(3-(Anilinomethylen)-2-chloro-1-cyclohexen-1-yl)-methylen]anilinhydro-chlorid in einem Gemisch aus 13 mL Butanol und 6 mL Toluen gelöst und 2 h bei 110 °C refluxiert Nach dem Abkühlen werden 92 mg (0,25 mmol) 1-(5-Acetoxypentyl)-2,3,3-trimethylindoleninium-bromid, hergestellt nach Hamilton et al., WO 97/40104, gelöst in 1,75 mL Butanol und 0,75 mL Toluen, hinzugetropft. Danach wird das Reaktionsgemisch über 10 h bei 110 °C refluxiert.

Anschließend wird das Lösungsmittel unter Vakuum abgezogen und der feste Rückstand mit Ether gewaschen. Es wird mit 20 mL 0,1 N HBr versetzt und 1 h unter Rückfluß erhitzt. Nun wird das Lösungsmittel unter Vakuum abrotiert und das Rohprodukt durch Chromatographie (Säulenmaterial Kieselgel, Eluent Methanol/Wasser 70/30 v/v) gereinigt.
Ausbeute: 23 mg (12%), grünes Pulver, C₃₉H₅₁BrClN₂O₄P (758,18 g/mol).

## Patentansprüche

1. Verbindungen nach der allgemeinen Formel (I), 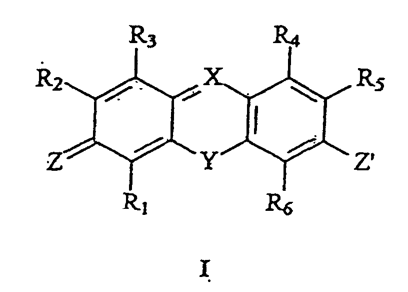 wobei der Rest X, der Rest C-R₇ oder N ist;
der Rest Y, der Rest NR₈, O, S, oder C(R₉)(R₁₀) ist;
der Rest Z, der Rest N(R₁₁)(R₁₂)^{⊕} oder O ist;
der Rest Z', der Rest OH oder N(R₁₃(R₁₄) ist;
wobei die Reste R, bis R₁₄ ausgewählt sind aus der Gruppe, bestehend aus H, Alkyl, Cycloalkyl, Aryl, Teilen eines aromatischen, heterocyclischen oder gesättigten, gegebenenfalls substituierten Ringsystems (gegebenenfalls mit dem Rest Z oder Z'), Alkoxy, Hydroxy, Nitro, Halogen, N(R₁₅)(R₁₆), und SR₁₇, wobei die Reste R₁₅, R₁₆ und R₁₇ die für die Reste R₁ bis R₁₄ angegebenen Bedeutung haben, wobei wenigstens einer der Reste R₁ bis R₁₇ eine reaktive Gruppe ist, die eine kovalente Bindung der Verbindung der Formel (I) mit einem Biomolekül oder Arzneimittel ermöglicht; oder nach einer der Formeln II, III, oder IV 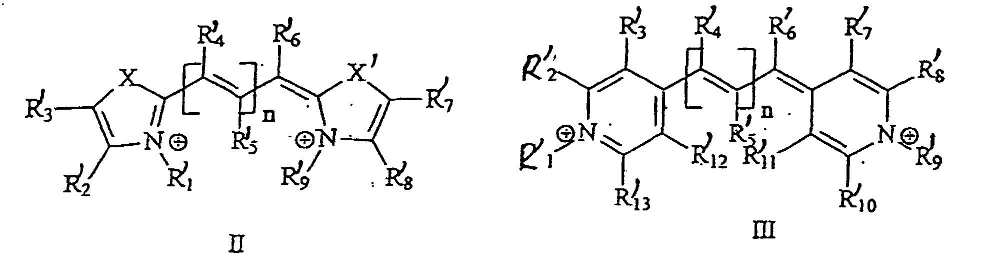 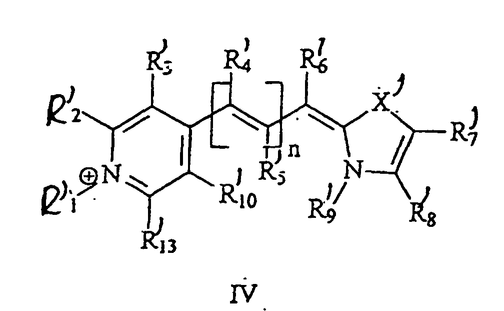 wobei der Rest X', ein Rest C(R'₁₄)(R'₁₅), N, O, S, Se oder CH=CH ist;
n 1, 2 oder 3 ist;
R'₁ bis R'₁₅ ausgewählt sind aus der Gruppe, bestehend aus H, Alkyl, Cycloalkyl, Aryl, Teilen eines armoatischen, heterocyclischen oder gesättigten, gegebenenfalls substituierten Ringsystems, Alkyoxy und Hydroxy, wobei
die Reste R'₄ und R'₅ auch zusammen eine Verbrückung über vier-, fünf-, und/oder sechsgliedrige Ringsysteme bilden können; und
wenigstens einer der Reste R₁' bis R₁₅' eine reaktive Gruppe ist, die eine kovalente Bindung der Verbindung der Formel (II), (III) oder (IV) mit einem Biomolekül oder Arzneimittel ermöglicht,
**dadurch gekennzeichnet, dass** wenigstens einer der Reste R₁ bis R₁₇ der Formel (I) oder einer der Reste R'₁ bis R'₁₅ der Formeln (II) bis (IV) ausgewählt ist aus der Gruppe, bestehend aus Phosphorsäure-, Phosphonsäure-Resten, deren Salzen und Monoestem.

2. Verbindungen nach Anspruch 1, wobei der wenigstens eine Rest ausgewählt aus der Gruppe, bestehend aus Phosphorsäure-, Phosphonsäure-Resten, deren Salzen und Monoestem direkt an das Doppelbindungssystem des Chromophors oder Fluorophors der Verbindungen der Formeln (I) bis (IV) gebunden ist

3. Verbindungen nach einem der Ansprüche 1 oder 2, wobei der wenigstens eine Rest ausgewählt aus der Gruppe, bestehend aus Phosphorsäure-, Phosphonsäure-Resten, deren Salzen und Monoestem über einen linearen oder verzweigten Spacer der allgemeinen Formel -(CH₂)ₙ- oder -[(CH₂)-O-(CH₂)ₘ]ₒ-, wobei n, m und o 1 bis 16 sind, an das Doppelbindungssystem des Chromophors oder Fluorophors der Verbindungen der Formeln (I) bis (IV) gebunden ist.

4. Verwendung einer Verbindung nach einem der Ansprüche 1 bis 3 als optischer Marker in fluoreszenzoptischen qualitativen oder quantitativen Bestimmungsverfahren oder Nachweistests.

5. Verwendung nach Anspruch 4, wobei die qualitativen oder quantitativen Bestimmungsverfahren oder Nachweistests dem Bestimmen oder Nachweisen von Biomolekülen oder Arzneimittel dienen.

## Claims

1. Compounds of the general formula (I) 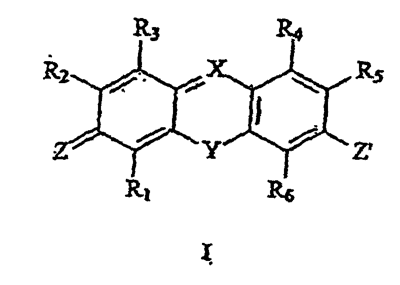 in which the residue X is the residue C-R₇ or N;
the residue Y is the residue NR₈, O, S or C(R₉)(R₁₀);
the residue X is the residue N(R₁₁)(R₁₂)⁺ or O;
the residue Z' is the residue OH or N(R₁₃)(R₁₄);
wherein the residues R₁ to R₁₄ are selected from the group comprising H, alkyl, cycloalkyl, aryl, parts of an aromatic, heterocyclic or saturated, optionally substituted ring system (optionally with the residue Z or Z'), alkoxy, hydroxyl, nitro, halogen, N(R₁₅)(R₁₆) and SR₁₇, the residues R₁₅, R₁₆ and R₁₇ having the meaning stated for the residues R₁ to R₁₄, at least one of the residues R₁ to R₁₇ being a reactive group which enables the compound of formula (I) to covalently bind to a biomolecule or drug; or of one of the formulae (II), (III) or (IV) 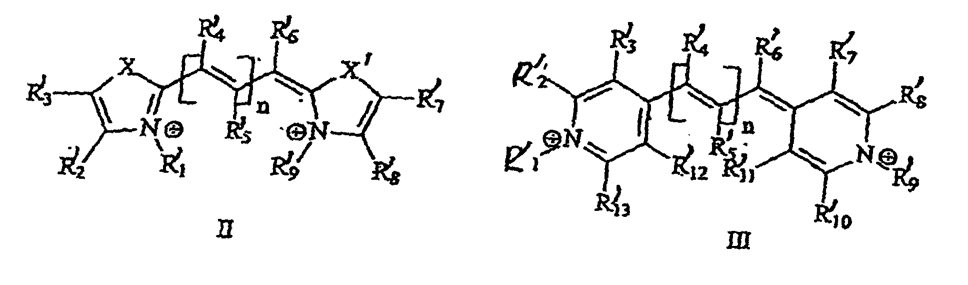 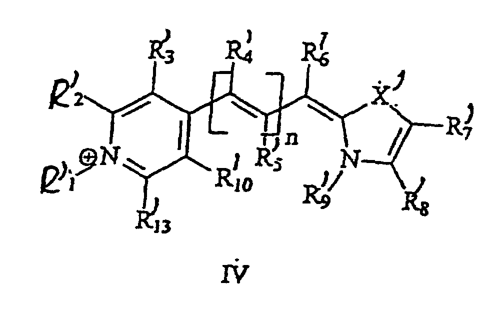 in which the residue X' is a residue C(R'₁₄)(R'₁₅), N, O, S, Se or CH=CH;
n is 1, 2 or 3;
R'₁ to R'₁₅ are selected from the group comprising H, alkyl, cycloalkyl, aryl, parts of an aromatic, heterocyclic or saturated, optionally substituted ring system, alkyloxy and hydroxy wherein
the residues R'₄ to R'₅ can also together form a bridge via four-, five- and/or six-membered ring systems; and
at least one of the residues R'₁ to R'₁₅ is a reactive group which enables the compound of formulae (II), (III) or (IV) to covalently bind to a biomolecule or drug,
**characterized in that** at least one of the residues R₁ to R₁₇ of formula (I) or one of the residues R'₁ to R'₁₅ of formulae (II) to (IV) are selected from the group comprising phosphoric acid, phosphonic acid residues, salts and monoesters thereof.

2. Compounds as claimed in claim 1, wherein the at least one residue selected from the group comprising phosphoric acid residues, phosphonic acid residues, salts and monoesters thereof is directly bound to the double bond system of the chromophore or fluorophore of the compounds of formulae (I) to (IV).

3. Compounds as claimed in one of the claims 1 or 2, wherein the at least one residue selected from the group comprising phosphoric acid residues, phosphonic acid residues, salts and monoesters thereof is bound to the double bond system of the chromophore or fluorophore of the compounds of formulae (I) to (IV) via a linear or branched spacer of the general formula -(CH₂)ₙ- or -[(CH₂)-O-(CH₂)ₘ]ₒ-, in which n, m and o are 1 to 16.

4. Use of a compound as claimed in one of the claims 1 to 3 as an optical marker in fluorescence optical, qualitative or quantitative determination methods or detection tests.

5. Use as claimed in claim 4, wherein the qualitative or quantitative determination method or detection test is used to determine or detect biomolecules or drugs.

## Revendications

1. Composés de la formule générale (I) 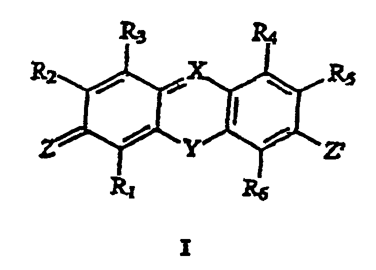 dans laquelle le reste X est le reste C-R₇ ou N ;
le reste Y est le reste NR₈, O, S ou C(R₉)(R₁₀) ;
le reste Z est le reste N(R₁₁)(R₁₂)^{⊕} ou O ;
le reste Z' est le reste OH ou N(R₁₃) (R₁₄) ;
dans lequel les restes R₁ à R₁₄ sont choisis dans le groupe consistant en H, alkyle, cycloalkyle, aryle, des parties d'un système cyclique aromatique, hétérocyclique ou saturé, éventuellement substitué (éventuellement avec le reste Z ou Z'), alcoxy, hydroxy, nitro, halogène, N(R₁₅) (R₁₆) et SR₁₇, dans lequel les restes R₁₅, R₁₆ et R₁₇ ont la signification indiquée pour les restes R₁ à R₁₄, dans lequel au moins l'un des restes R₁ à R₁₇ est un groupe réactif qui permet une liaison par covalence du composé de la formule (I) avec une biomolécule ou un médicament ; ou selon l'une des formules (II) , (III) ou (IV) 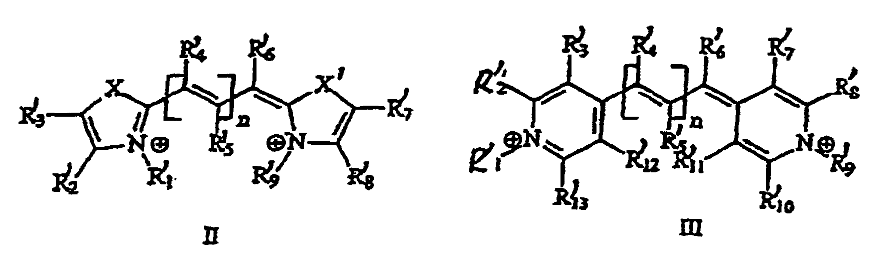 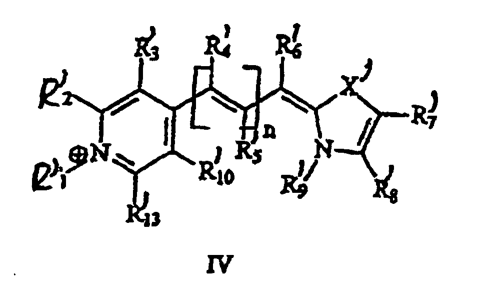 dans lesquelles le reste X' est un reste C(R'₁₄)(R'₁₅), N, O, S, Se ou CH=CH ;
n est égal à 1, 2 ou 3 ;
R'₁ jusqu'à R'₁₅ sont choisis dans le groupe constitué par H, alkyle, cycloalkyle, aryle, des parties d'un système cyclique aromatique, hétérocyclique ou saturé, éventuellement substitué, alcoxy et hydroxy, dans lequel les restes R'₄ et R'₅ peuvent également former conjointement une liaison par l'intermédiaire de système cyclique par quatre, cinq et/ou six chaînons ; et
au moins l'un des restes R₁' à R₁₅' est un groupe réactif qui permet une liaison par covalence du composé des formules (II), (III) ou (IV) avec une biomolécule ou un médicament,
**caractérisé en ce qu'**au moins l'un des restes R₁ à R₁₇ de la formule (I) ou l'un des restes R'₁ jusqu'à R'₁₅ des formules (II) à (IV) est choisi dans le groupe constitué par l'un des restes d'acide phosphorique, d'acide phosphonique, leurs sels et monoesters.

2. Composés selon la revendication 1, dans lesquels le au moins un reste choisi dans le groupe constitué par les restes d'acide phosphorique, acide phosphonique, leurs sels et monoesters est lié directement au système à double liaison du chromophore ou fluorophore des composés des formules (I) à (IV).

3. Composés selon l'une des revendications 1 ou 2,
dans lequel le au moins un reste choisi dans le groupe, constitué par les restes d'acide phosphorique, acide phosphonique, leurs sels et monoesters est lié par l'intermédiaire d'un spacer ou bras espaceur. linéaire ou ramifié de la formule générale -(CH₂)ₙ- ou -[(CH₂)-O-(CH₂)ₘ]ₒ-, dans lalquelle n, m et o sont 1 jusqu'à 16, et sont liés au système double liaison du chromophore ou fluorophore des composés des formules (I) à (IV).

4. Utilisation d'un composé selon l'une des revendications 1 à 3 en tant que marqueur optique dans des procédés d'analyse ou des tests de détection qualitatifs ou quantitatifs par fluorescence optique.

5. Utilisation selon la revendication 4, dans laquelle les procédés d'analyse ou tests de détection qualitatifs ou quantitatifs par fluorescence optique servent à identifier ou à détecter des biomolécules ou des médicaments.
